# EUROPEAN PATENT SPECIFICATION

(11) **EP 4 196 461 B1**
(45) Date of publication and mention of the grant of the patent: **20.08.2025**
(21) Application number: 21765714.7
(22) Date of filing: 13.08.2021
(51) Int. Cl.: C07C 229/12, C07C 229/20, C07C 229/36, C07C 323/58, C07D 209/20, C11D 1/62, A61K 8/45, A61Q 5/02, A61Q 5/12, A61Q 19/00

(54) **QUATERNARY AMMONIUM SALT FOR USE AS A CATIONIC SURFACTANT**
QUATERNÄRES AMMONIUMSALZ ZUR VERWENDUNG ALS KATIONISCHES TENSID
SEL D'AMMONIUM QUATERNAIRE DESTINÉ À UNE UTILISATION COMME TENSIOACTIF CATIONIQUE

(30) Priority: 17.08.2020 IT 202000020212; 27.01.2021 IT 202100001586; 13.04.2021 IT 202100009233
(43) Date of publication of application: 21.06.2023
(73) Proprietor: Greengredients S.r.l., 05100 Terni (TR) (IT)
(72) Inventor: BREGAGLIO, Guido, 27020 Torre d'Isola PV (IT)
(74) Representative: Perani & Partners S.p.A.
(86) International application number: PCT/IB2021/057481
(87) International publication number: WO 2022/038477

(56) References cited:
- WO-A2-2006/010647
- WO-A2-2012/021436
- FR-A1- 2 926 979
- JP-A- 2002 275 759
- JP-A- 2005 170 886
- US-B1- 8 273 920
- KITSUKI ET AL: "Fiber treatment agent composition with excellent detergency for cleaning stains on textile products", CA, CHEMICAL ABSTRACTS SERVICE, COLUMBUS, OHIO, US, 2002 - 2002, XP002802765

## Description

### FIELD OF THE INVENTION

The field of the present invention concerns a quaternary ammonium salt that can be used as a green alternative to common cationic surfactants.

### BACKGROUND

According to IUPAC, the International Union of Pure and Applied Chemistry, green chemistry means "the invention, design and use of chemicals and processes for reducing or eliminating the use and the production of hazardous substances". The main objectives of green chemistry include: preventing the production of waste, rather than treating it after it has been produced; minimising the toxicity of the chemicals used; and using renewable raw materials wherever possible.

Quaternary ammonium salts are widely used as cationic surfactants for fabric softeners, anti-electrostatic agents, corrosion inhibitors, hair conditioners, dispersants, germicides, biocides.

Among these quaternary ammonium salts, cetrimonium chloride or behentrimonium chloride are known and are mainly used as cationic surfactants in cosmetic and/or household products. Other types of quaternary ammonium salts acting as cationic surfactants and biocides are the polymers of quaternary ammonium salts such as, for example, silicone cocoamido quat or silicone dimeramido quat.

The products potentially containing quaternary ammonium salts as cationic surfactants are usually used on a daily and/or high frequency basis by consumers. In addition, they come into direct contact with the consumer's skin layer and/or are sometimes inhaled in the form of vapours.

WO2006/010647 describes an ester of a monoglycerol with a quaternary ammonium salt which could potentially derive from an amino acid (an "α-carbon" is identifiable, located between the polyglycerol ester and the quaternary nitrogen, which is typical of amino acid structures). It also addresses the issue of the environmental sustainability of the surfactant, the molecule, in fact, being degradable in water, is presented to have a reduced environmental impact. Particularly, it discloses a cosmetic composition for cleaning and/or conditioning "human keratin fibres" (i.e. hair), comprising at least one cationic surfactant.

JP2005170886 describes a quaternary ammonium salt having a chain comprising an alkylated derivative of polyethylene glycol, wherein the counteranion X⁻ is a halogen (Cl-, Br-, I-).

FR2926979 describes a quaternary ammonium salt with a chain including an acylated polyethylene glycol derivative. The counter anions for neutralisation of the salt are chosen from: acetate, citrate, tartrate, halogen, SO₄²⁻ , MeSO₄⁻ , EtSO₄⁻ . The product is included in a cosmetic composition intended for cleansing and care of the skin of the body or face; lips; eyebrows; eyelashes; nails and hair. Or, in a composition for hair styling, hair colouring.

US8273920 discloses a quaternary ammonium salt with a chain comprising an alkyl derivative of polyethylene glycol having an alkane group.

WO2012/021436 discloses a salt having the quaternary ammonium group in the middle position, and two chains comprising an acyl derivative of polyethylene glycol.

Kitsuki, Tomohito et al., "Fiber treatment agent composition with excellent detergency for cleaning stains on textile products" (XP002802765) discloses a molecular structure of an amino-acid-derived quaternary ammonium salt; an α-carbon can be identified between the quaternary ammonium group and the ester group.

### Problem of the prior art

The common quaternary ammonium salts used as cationic surfactants are associated with certain drawbacks, such as:
- are hardly or not at all biodegradable and therefore constitute a potential toxic waste, together with their chemical derivatives, if released into the environment;
- are generally characterised by an unpleasant smell that makes them difficult to use. This becomes particularly relevant when they are included in cosmetic and/or personal cleansing products. For example, quaternary ammonium chloride salts are often characterised by a rotten fishy smell.

In addition, one of the common synthetic processes for quaternary ammonium salts is based on the Menshutkin reaction (or *quaternization reaction*); the latter involves the use of polar solvents (e.g. methanol, ethanol) and of an alkyl halide for the alkylation of the tertiary amine group; these alkyl halides are commonly synthesised in the laboratory using toxic solvents and/or reagents (e.g. in the presence of pyridine) - a factor that negatively contributes to the eco-sustainability of these salts.

Furthermore, when used in personal cleansing and cosmetic products, they tend to accumulate at the skin level (mainly cetrimonium chloride or CTAC) and to cause damage to the most superficial skin layer, promoting the onset of skin irritation and/or sensitisation phenomena, especially if used on a high frequency basis; they are sometimes responsible for inducing dehydration phenomena of the skin layer.

### SUMMARY OF THE INVENTION

The Applicant has synthesised a quaternary ammonium salt that solves the problems of the prior art. The object of the present invention is a quaternary ammonium salt of formula (I) 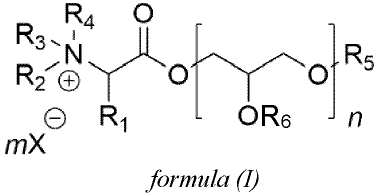 wherein the substituents R₅is chosen between hydrogen and a radical R₀ wherein the radical R₀ consists of the following structure of formula (II) 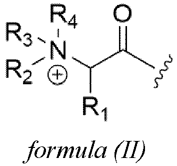 the substituent R₆ is hydrogen,
wherein the substituent R₁ is hydrogen,
wherein the substituents R₂, R₃, R₄ are methyl,
wherein the counteranion *m*X⁻ is chosen from the group of carboxylic acids consisting of: formic acid, acetic acid, unsaturated monocarboxylic acids, tartaric acid, adipic acid, aldaric acid, oxalic acid, phthalic acid, azelaic acid, sebacic acid, malonic acid, succinic acid, glutaric acid, pimelic acid, maleic acid, malic acid, fumaric acid, suberic acid, citric acid, isocitric acid, fatty acids, amino acids, keto acids and aromatic carboxylic acids,
wherein *m* is an integer number comprised between 1 and 22,
wherein n is comprised between 2 and 20.

Further object of the present invention are compositions comprising the quaternary ammonium salt of formula (I) as a cationic surfactant. Said compositions may preferably be cosmetic type compositions or cleaning type, softening/conditioning compositions for surface treatment.

### Advantages of the invention

The quaternary ammonium salt of formula (I) developed by the Applicant is advantageous over common quaternary ammonium salts (quat) because:
- it is biodegradable and non-toxic, thus making it a green alternative to the ordinary quat;
- it can be synthesised without using toxic reagents and/or solvents, heavy metals, petrochemical derivatives or genetically modified organisms (GMOs);
- it is available in liquid form for easier solubilisation/dispersion in commercial product compositions;
- it is characterised by a more pleasant smell compared to that of the common quaternary ammonium salts, the counteranion of which is commonly a halide, mainly chloride. Specifically, the use of an organic counteranion X⁻ makes it possible to obtain a salt of formula (I) characterised by a pleasant smell;
- the organic counteranion (*m*X⁻), plays a dual role: on the one hand, it acts as a counteranion neutralising the quaternary ammonium salt of formula (I); on the other hand, it acts as an acid catalyst during the synthesis of the quaternary ammonium salt of formula (I), for the esterification reaction and for obtaining the desired product. In this sense, the salt of the invention is made without the aid of catalysts based on heavy metals that can create toxicity problems in the final product.

The quaternary ammonium salt of formula (I) can advantageously be used as a cationic surfactant in cosmetic type and personal cleansing compositions. For these purposes it is advantageous because:
- it is able to moisturise the skin layer;
- it is not toxic, irritating or sensitising to the skin layer;
- it does not accumulate in the skin layer;
- it is suitable for palm-free cosmetic formulations/compositions;
- when used in hair care products, it facilitates the operation of combing the hair when wet and of detangling the hair when dry while reducing the electrostatic fly-away phenomenon of the hair;
- it can be used as the sole cationic surfactant in hair cosmetic compositions, showing comparable or more advantageous effects than common surfactants (see section Examples); on the other hand, it can be used in association with common cationic surfactants;
- it acts as a stabiliser for emulsions/solutions and it is also compatible with other non-ionic and/or negatively charged ingredients;
- the use of the cosmetic product comprising the salt of formula (I) does not require a hot process.

In addition, as mentioned above, the quaternary ammonium salt of formula (I) can advantageously be used as a cationic surfactant in compositions for surface treatment, e.g. for household cleaning or cleaning of surfaces in general; in formulations for cleaning fabrics and leathers, in particular in products with softening power. For these purposes it is advantageous because:
- it is not toxic, irritating or sensitising to the skin layer;
- when used for fabric washing, the quaternary ammonium salt of formula (I) has a softening power and at the same time a gentle cleaning power that does not ruin fabrics;
- it reduces the static charge of the surfaces; this effect is particularly desirable in fabric washing operations, since it gives the garment a greater softness;
- it can be used as the sole cationic surfactant or in association with common cationic surfactants;
- it acts as a stabiliser for emulsions/solutions and it is also compatible with other non-ionic and/or negatively charged ingredients;
- the preparation of compositions/formulations including the salt of formula (I) does not require a hot process.

### DESCRIPTION OF THE DRAWINGS

Figure 1 - Synthesis scheme of the salt of formula (I)
Figure 2 - Tensiometer instrument (INSTRON Mod. 5543)
Figure 3 - Comparison between strand of hair treated according to example 2.1. Lefthand strand: the hair is smoother and more moisturised to the touch; healthier to the eye. A reduction in the frizz and fly-away effect is also visible; right-hand strand: the hair is more frizzy and the onset of the fly-away effect is visible.
Figure 4 - Measurement of dry detangling of hair.
Figure 5 - Measurement of the detangling capacity on damaged Caucasian and Chinese hair, treated with a 5% aqueous solution of salt of formula (I_{A1}) and an equal amount of a 5% aqueous solution of a 50:50 combination of CTAC/BTAC.
Figure 6 - Measurement of the thickness assessed on five hair strands after 20 brush strokes.
Figure 7 - Measurement of the hair breakage after 10 brush strokes.
Figure 8 - Method for measuring the electrostatic charge.

### DETAILED DESCRIPTION OF THE INVENTION

### Salt of formula (I)

With reference to formula (I), the invention will be described hereinbelow in more detail, referring where necessary to the preferred embodiment of the invention.

The substituent R₁ of the salt of formula (I) is hydrogen.

It should be noted that, depending on whether the substituents R₅ and R₆ are equal to hydrogen (-H) or to the radical R₀ (formula II), the value of *m* may change accordingly.

In general, *m* with reference to the counteranion X⁻ represents the number of molecules of the counteranion contained in the salt of formula (I). Preferably, *m* is a number such that it guarantees the neutrality of the molecule of the salt of formula (I). In particular, *m* is an integer number comprised between 1 and 22, preferably comprised between 1 and 12, preferably comprised between 1 and 8, preferably equal to 1 or 2 or 3 or 4 or 5 or 6 or 7.

According to a particularly preferred embodiment, the salt of formula (I), comprises, at most, a number of quaternary ammonium groups equal to 3, irrespective of the value of *n.* In other words, the quaternary ammonium molecule and its counteranion X⁻ are preferably between them in a stoichiometric ratio equal to 1: 1 or alternatively equal to 1:2 or alternatively equal to 1:3, regardless of the value of *n.*

The following Table 1 shows the structural formulas of the different preferred embodiments of the salt of formula (I) for each sub stituent R₁ not according the present invention for which R₁ is hydrogen.

**Table 1**

| **R₁** | **Formula (name)** | **Structure** |
|---|---|---|
| hydrogen | (I_{A}) | 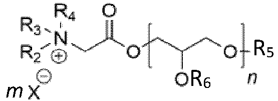 |
| methyl | (I_{B}) | 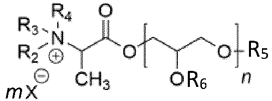 |
| Isopropyl | (I_{C}) | 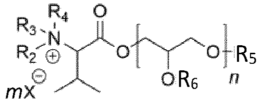 |
| Isobutyl | (I_{D}) | 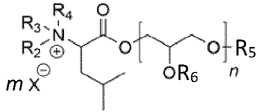 |
| Sec-butyl | (I_{E}) | 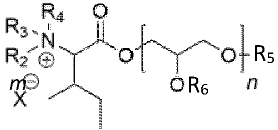 |
| ethylenemethylthio | (I_{F}) | 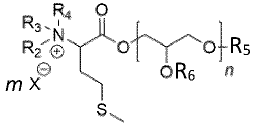 |
| Benzyl | (I_{G}) | 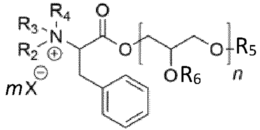 |
| Para-hydroxybenzyl | (I_{H}) | 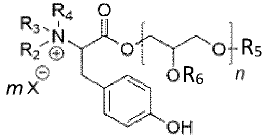 |
| 3-methylene-*1H-*indole | (I_{I}) | 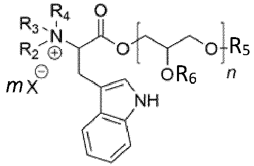 |

R₁ is hydrogen; in other words, the preferred embodiment of the salt of formula (I) is (I_{A}).

For the embodiment indicated in Table 1 (I_{A}), the substituents R₂, R₃, R₄ are methyl.

The substituents R₂, R₃ and R₄ of the salt of formula (I) are equal to each other and are methyl.

The substituents R₂, R₃ and R₄ of the salt of formula (I) are equal to each other and consist of the linear methyl substituent.

The substituents R₂, R₃ and R₄ of the salt of formula (I_{A}) are equal to each other and consist of the linear methyl substituent.

It should be noted that the considerations made above with reference to the substituents R₂, R₃, R₄ apply to both the quaternary ammonium group of the salt of formula (I) and to the radical R₀.

For the embodiment indicated in Table 1 (I_{A}), the counteranion *m*X⁻ is chosen from the group of carboxylic acids (in the form of carboxylate ions) consisting of:
- monocarboxylic acids, preferably chosen between acetic acid and formic acid;
- unsaturated monocarboxylic acids, among which the acrylic acid is preferred one;
- bicarboxylic (or dicarboxylic) acids, preferably chosen from the group consisting of: adipic acid, aldaric acid, oxalic acid, phthalic acid, azelaic acid, sebacic acid, malonic acid, succinic acid, tartaric acid, glutaric acid, pimelic acid, maleic acid, malic acid, fumaric acid, suberic acid;
- tricarboxylic acids, preferably chosen between citric acid and isocitric acid;
- fatty acids, preferably chosen from the group consisting of butyric acid, oleic acid, palmitic acid, stearic acid;
- acid amino acids, preferably chosen from the group consisting of glutamic acid and aspartic acid;
- keto acids, preferably chosen between: acetoacetic acid, pyruvic acid and levulinic acid; and
- aromatic carboxylic acids, preferably chosen from the group consisting of benzoic acid, salicylic acid, cinnamic acid, caffeic acid.

It should be noted that fatty acids are defined as saturated or unsaturated aliphatic monocarboxylic acids with a number of carbon atoms ≥ 4 and ≤ 20, preferably ≥ 4 and ≤ 18.

For the purposes of the present invention, the keto acids are carboxylic acids which contain a ketone residue and which are involved in various biological processes, such as, for example, the process of forming ketone bodies or of glycolysis.

For the embodiment (I_{A}) of the quaternary ammonium salt of the invention, the counteranion X⁻ is preferably chosen from the group of carboxylic acids (in the form of carboxylate ions) consisting of: formic acid, acetic acid, acrylic acid, adipic acid, aldaric acid, oxalic acid, phthalic acid, azelaic acid, sebacic acid, malonic acid, succinic acid, tartaric acid, glutaric acid, pimelic acid, maleic acid, malic acid, fumaric acid, suberic acid, citric acid, isocitric acid, butyric acid, oleic acid, palmitic acid, stearic acid, glutamic acid, aspartic acid, acetoacetic acid, pyruvic acid, levulinic acid, benzoic acid, salicylic acid, cinnamic acid, caffeic acid.

A polyglycerol-*n* is defined as a polymer consisting of an *n* number of structural-base units of glycerol; it should be noted that the commonly commercially available polyglycerols-*n* are mixtures of polyglycerols, comprising 60% or more of the polyglycerol-*n* of interest in which about 20% of the mixture consists of one or more of its different homologues, i.e., polyglycerols-*n* with a lower or higher *n* number of repeating units than the one considered.

In particular, a polyglycerol-n in which *n* ≤ 10 can be synthesised using green experimental methods starting from natural plant glycerine, following the *Cosmos* and *NaTrue* standards.

In contrast, a polyglycerol-*n* in which *n* > 10 is normally obtained by experimental methods known to the person skilled in the art which include the use of synthetic (nonvegetable) glycerin.

According to a preferred embodiment of the quaternary ammonium salt of formula (I), *n* is comprised between 2 and 10, preferably comprised between 2 and 6, preferably equal to 2, 3, 4 or 5.

In solution (or mixture), the quaternary ammonium salt of formula (I) is preferably electrically neutral. For the purposes of the present invention, electrically neutral means a molecular structure that contains an equal number of positive charges and of negative charges.

### Embodiments in which the substituent R₅ is hydrogen and the substituent R₆ is hydrogen

According to a preferred embodiment of the salt of formula (I), preferably of the salt of formula (I_{A}), the substituent R₅ is equal to hydrogen H and the substituent R₆ is hydrogen.

In this preferred embodiment, the salt of formula (I), preferably the salt of formula (I_{A}), is characterised by a single positive charge on the quaternary nitrogen *N*.

According to this preferred embodiment, the value of *m* is preferably equal to 1. Still preferably, in this first preferred embodiment, the positive charge of the quaternary nitrogen is neutralised by the counteranion *m*X⁻, which satisfies the following conditions
- *m* is equal to 1 and X⁻ is a monoprotic carboxylic acid (or monocarboxylic acid), preferably it is the acetic acid;
   or alternatively
- *m* is equal to 1 and X⁻ is a pluriprotic carboxylic acid but with *only* one ionisable/ionised carboxylic group, preferably it is the malic acid or the citric acid or the tartaric acid.

It should be noted that, preferably, the degree of ionisation of the pluriprotic carboxylic acid is influenced by the pH conditions of the chemical environment in which the salt of formula (I) is placed, preferably the salt of formula (I_{A}), or to those of the process at which the quaternisation thereof takes place. In fact, although the quaternisation reaction carried out under acid catalysis is advantageous because it uses the carboxylic acid destined to neutralise the quaternary *N*, the same reaction can also be carried out under alkaline catalysis.

### Embodiments in which the substituent R₅ is equal to the radical R₀ and the substituent R₆ is hydrogen

According to a preferred embodiment of the salt of formula (I), preferably of the salt of formula (I_{A}), the substituent R₆ is hydrogen, the substituent R₅ is equal to the radical R₀, characterised by the following structure of formula (II):

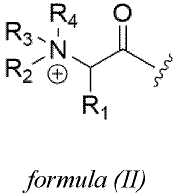

According to this embodiment, the substituent R₁ of R₀ is hydrogen.

R₁ of R₀ is hydrogen; for the substituents R₂, R₃, R₄, the above considerations apply.

The substituents R₂, R₃ and R₄ of R₀ are equal to each other and consist of the linear methyl substituent.

According to this preferred embodiment, the salt of formula (I), preferably of formula (I_{A}), is characterised by a double positive charge on the corresponding quaternary nitrogen atoms *N*.

According to this preferred embodiment, the value of *m* may preferably be equal to 1 or 2. Still preferably, the positive charges of the quaternary nitrogen atoms are neutralised under the following conditions:
- *m* is equal to 1 and X⁻ is a totally ionised diprotic carboxylic acid (or dicarboxylic acid), preferably it is the malic acid or the tartaric acid;
- *m* is equal to 1 and X⁻ is a triprotic carboxylic acid (or tricarboxylic acid) with *only two* ionisable/ionised carboxylic groups, preferably it is the citric acid;
- *m* is equal to 2 and X⁻ is a monoprotic carboxylic acid, preferably it is the acetic acid; or
- *m* is equal to 2 and X⁻ is a pluriprotic carboxylic acid with *only one* ionisable/ionised carboxylic group, preferably it is the malic acid, the tartaric acid or the citric acid or combinations of the foregoing.

Still preferably, when the substituent R₅ is equal to R₀ and *m* is equal to 2, the two counteranions X⁻ neutralising the two positive charges of the nitrogen atoms of the salt of formula (I), preferably of formula (I_{A}), may consist of the same carboxylic acid (same entity) or of two carboxylic acids of different nature (different entity).

The Applicant hereinafter describes particularly preferred embodiments of the salt of formula (I_{A}).

### Embodiments in which the substituent R₅ and the substituent R₆ are equal to the radical R₀, not part of the present invention

According to a embodiment of the salt of formula (I) not covered by the present invention, preferably of the salt of formula (I_{A}), the substituents R₆ and R₅ are equal to the radical R₀ (formula II) and equal to each other.

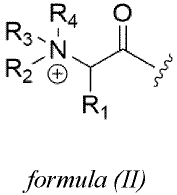

According to this embodiment not covered by the present invention, the substituent R₁ of R₀ is selected from the group consisting of: hydrogen, methyl, isopropyl, *sec*-butyl, isobutyl, ethylenemethylthio, benzyl, *para*-hydroxybenzyl and 3-methylene-*1H-*indole.

Preferably, R₁ of R₀ is hydrogen; for the substituents R₂, R₃, R₄, the above considerations apply.

According to a particularly preferred embodiment mode of the present embodiment not covered by the present invention, the substituents R₂, R₃ and R₄ of R₀ are equal to each other and consist of the linear methyl substituent.

According to this preferred embodiment not covered by the present invention, the salt of formula (I), preferably of formula (I_{A}), is characterised by a plurality of positive charges on the corresponding quaternary nitrogen atoms N.

Still preferably, the salt of formula (I), preferably of formula (I_{A}), is characterised by a maximum of 3 quaternary ammonium groups and thus by three positive charges on the corresponding quaternary nitrogen atoms *N,* irrespective of the value of *n.*

According to this preferred embodiment not covered by the present invention, the value of *m* may preferably be equal to 1 or 2 or 3. Still preferably, the positive charges of the quaternary nitrogen atoms are neutralised under the following conditions:
- *m* is equal to 1 and X⁻ is a totally ionised triprotic carboxylic acid (or tricarboxylic acid), preferably it is the citric acid;
- *m* is equal to 2 and X⁻ is a mixture of a monoprotic carboxylic acid (or monocarboxylic acid) and a totally ionised diprotic carboxylic acid (or dicarboxylic acid), preferably chosen between acetic acid, malic acid, citric acid, tartaric acid and combinations of the foregoing;
- *m* is equal to 2 and X⁻ is a partially ionised pluriprotic carboxylic acid, preferably chosen from acetic acid, malic acid, citric acid, tartaric acid and combinations of the foregoing;
- *m* is equal to 3 and X⁻ is a monoprotic carboxylic acid, preferably it is the acetic acid; or
- *m* is equal to 3 and X⁻ is a pluriprotic carboxylic acid with *only one* ionisable/ionised carboxylic group, preferably it is the malic acid, the tartaric acid or the citric acid or combinations of the foregoing.

Still preferably, when the substituent R₅ is equal to R₀ and *m* is equal to 2, the two counteranions X⁻ neutralising the two positive charges of the nitrogen atoms of the salt of formula (I), preferably of formula (I_{A}), may consist of the same carboxylic acid (same entity) or of two carboxylic acids of different nature (different entity).

The Applicant hereinafter describes particularly preferred embodiments of the salt of formula (I_{A}).

### - First preferred embodiment of the salt of formula (I_{A})

According to a first preferred embodiment of the salt of formula (I_{A}), the substituents R₅ and R₆ are both hydrogen, *m* is equal to 1, the substituent R₁ is hydrogen, the substituents R₂, R₃ and R₄ are methyl, as well as in the following formula:

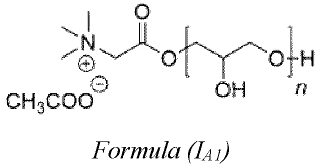

Preferably *n* is equal to 2, 3, 4 or 5.

According to this first preferred embodiment, the counteranion X⁻ is acetate. The acetate counteranion is particularly advantageous for the purposes of the invention because it gives the quaternary ammonium salt of formula (I) a pleasant fruity smell.

### - Second preferred embodiment of the salt of formula (I_{A})

According to a further preferred embodiment of the salt of formula (I_{A}), the substituents R₅ and R₆ are both hydrogen, *m* is equal to 1, the substituent R₁ is hydrogen, the substituents R₂, R₃ and R₄ are methyl and the counteranion X⁻ is malate, as well as in the following formula (I_{A2}):

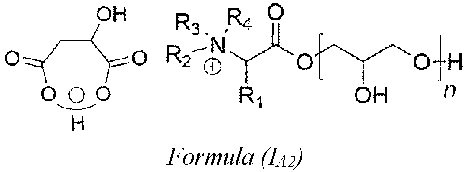

Preferably *n* is equal to 2, 3, 4 or 5.

The Applicant points out that the malate ion as depicted in formula (I_{A2}) is for illustrative purposes only and not limiting the purposes of the invention.

For the purposes of the present invention, the malate is preferably the dicarboxylate ion of the natural enantiomer of the malic acid, namely the *L*-form (or 2*S*-hydroxy-1,4-butanedioic acid), which is the one biologically active.

### - Third preferred embodiment of the salt of formula (I_{A})

A third embodiment is the quaternary ammonium salt characterised by the following structure formula (I_{A3}) , wherein the substituent R₅ is equal to the radical R₀, R₆ is hydrogen, R₁ is hydrogen, the substituents R₂, R₃ and R₄ are methyl groups, wherein *m* is equal to 2 and the counteranion X⁻ is acetate:

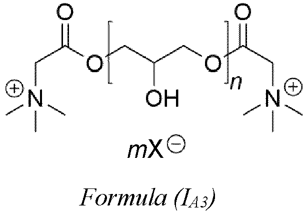

Preferably n is equal to 2, 3, 4 or 5.

According to an alternative embodiment of the salt of Formula I_{A3} X⁻ is the monoionised malate ion or the monoionised tartrate ion and *m* is equal to 2.

### - Fourth preferred embodiment of the salt of formula (I_{A})

A fourth embodiment of the invention is the quaternary ammonium salt characterised by the following structure formula (I_{A4}) , wherein the substituent R₅ is equal to the radical R₀, R₆ is hydrogen, R₁ is hydrogen, the substituents R₂, R₃ and R₄ are methyl groups, wherein *m* is equal to 1 and the counteranion X⁻ is the malate ion or the tartrate ion:

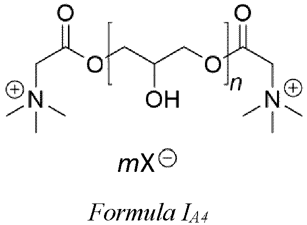

According to this fourth embodiment of structure formula (I_{A4}), *n* is equal to 2, 3, 4, or 5.

### - Fifth preferred embodiment of the salt of formula (I_{A}), not covered by the present invention

A fifth embodiment not covered by the invention is the quaternary ammonium salt characterised by the following structure formula (I_{A5}), wherein the substituents R₅ and R₆ are equal to the radical R₀, R₁ is hydrogen, the substituents R₂, R₃ and R₄ are methyl groups, wherein *m* is equal to 1 or 3 and the counteranion X⁻ is the citrate ion or the acetate ion:

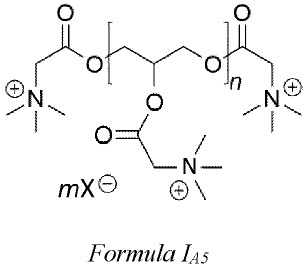

According to this fourth embodiment of structure formula (I_{A4}), *n* is equal to 2, 3, 4, or 5. Still preferably, the number of quaternary ammonium groups is equal to 3, regardless of the *n* value.

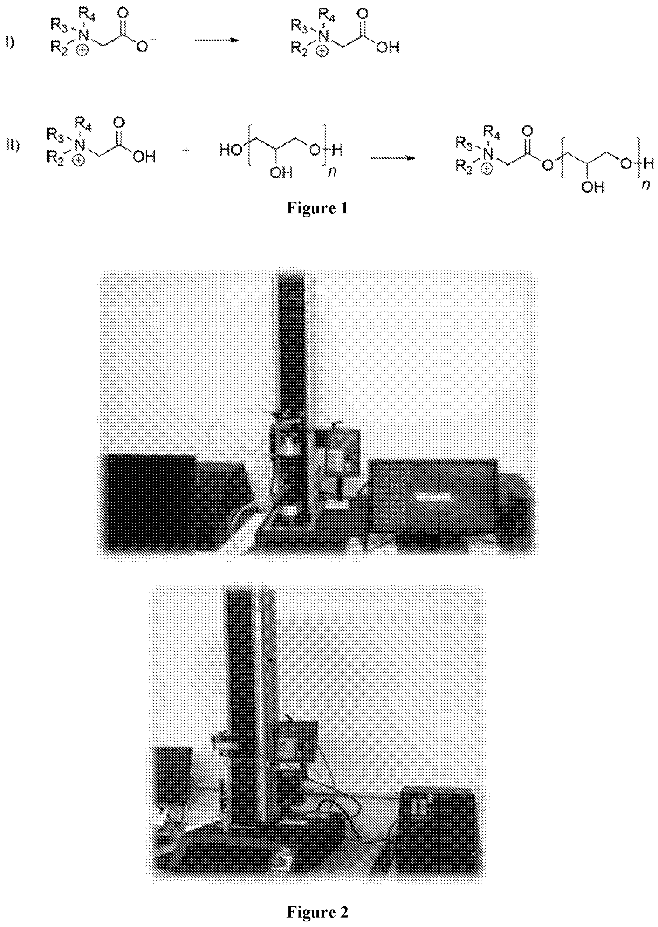

The quaternary ammonium salt of formula (I) can be advantageously obtained using the reactions known in the state of the art: by way of example, Figure 1 shows the synthesis scheme of the quaternary ammonium salt of formula (I_{A}) starting from the trimethylglycine acidification reaction (reaction I, Figure 1), and subsequent organic acid catalysed esterification reaction with the polyglycerol-*n* reagent (reaction II, Figure 1). In this way, the desired quaternary ammonium salt product of formula (I_{A}) and water are obtained.

It should be noted that, advantageously, water is the *only* by-product of the synthesis reaction of the quaternary ammonium salt of formula (I); thus, there is no formation and/or potential release of other compounds or chemical derivatives into the environment.

As with the quaternary ammonium salt of formula (I_{A}), the synthesis reaction of the quaternary ammonium salt of formula (I) can be carried out starting from amino acid derivatives characterised by a quaternary amine group at carbon α (*N-R₂, R₃, R₄*)*.*

Advantageously, the starting amino acid derivative (*N-R₂, R₃, R₄*)*,* the polyglycerol-*n* and the organic acid used in these chemical reactions are in turn obtained, as far as possible, by green synthesis methods according to the *Cosmos* and *NaTrue* standards and the Regulation (EC) No. 1223/2009, thus contributing to the sustainability of the final product.

### Composition comprising at least one salt of formula (I) as a cationic surfactant

The salt of formula (I), preferably of formula (I_{A}), is preferably used as a cationic surfactant.

Preferably, the salt of formula (I) is used as a wetting agent, conditioning/softening, emulsifying, cleaning, antistatic agent.

As already anticipated, further object of the present invention is a composition comprising at least one quaternary ammonium salt formula (I) as a cationic surfactant, in combination with possible excipients and/or diluents.

In the composition claimed preferably the salt of formula (I) is in a concentration comprised between about 0.25% and 7% (w/w), preferably between about 0.25% and 5%, preferably between about 0.5% and 5% (w/w), preferably between about 1% and 5% (w/w), preferably equal to about 0.25%, 0.5%, 1%, 2%, 3%, 4%, 5% (w/w) by weight on the total weight of the composition.

In the composition comprising the salt of formula (I), the latter may be the sole cationic surfactant or may be in combination with other cationic surfactants.

When used in combination with other cationic surfactants (e.g. polymeric cationic surfactants), at the dosages indicated above, the salt of formula (I) enables the use of silicone derivatives to be avoided or the use of other polymeric cations to be reduced.

Cationic surfactants that can be used in combination with the salt of formula (I) are, for example, chosen from the group consisting of: cetrimonium chloride (or CTAC), behentrimonium chloride (or BTAC), stearamidopropyl dimethylamine (or SAPDMA) and guar hydroxypropylthrimethylammonium chloride (or GHPTAC), Dioleylethyl Hydroxyethylmonium Methosulfate (Tetranil CO-40) and Behenoyl PG-Trimonium Chloride (Quartamina BTC-131).

The composition preferably has a pH higher than 3.5 and less than 6.0; preferably the cosmetic composition has a pH comprised between 3.5 and 5, preferably comprised between 4 and 4.5.

The pH of the composition can be regulated by including one or more buffering agents in the composition; an average person skilled in the art, on the basis of his basic knowledge, would be able to select without any difficulty one or more suitable buffering agents for the purpose, choosing among those known to be state of the art.

### Cosmetic composition comprising the salt of formula (I)

According to an embodiment of the invention, the composition comprising the salt of formula (I), preferably of formula (I_{A}), is of the cosmetic type.

The cosmetic composition comprising the salt of formula (I) may further comprise suitable excipients and/or diluents known to the person skilled in the art. By way of example, suitable excipients and/or diluents are selected among: water; cationic surfactants; anionic surfactants; amphoteric surfactants; conditioning agents; active ingredients, e.g. proteins, amino acids, osmoprotectants, vitamins; anti-dandruff agents; thickening agents; viscosity agents; filming agents, emulsifiers; antioxidants; chelators; pigments for hair colouring; buffering agents for pH regulation; natural extracts; fragrances; essential oils; humectants.

The cosmetic composition preferably has a pH higher than 3.5 and less than 6.0; preferably a pH comprised between 3.5 and 5, preferably comprised between 4 and 4.5.

Without wishing to be bound by any theory, the Applicant believes that the pH influences the conditioning capacities of a cationic system; in this sense, at the above-mentioned pH values, the cosmetic composition has shown better conditioning capacities: the hair could be more easily combed than at other pH values of the composition.

The pH of the cosmetic composition can be regulated by including one or more buffering agents in the composition; an average person skilled in the art, on the basis of his basic knowledge, would be able to select without any difficulty one or more suitable buffering agents for the purpose, choosing among those known to be state of the art.

The cosmetic (or cosmetic type) composition is preferably in the form of a cleansing product for the skin and hair, a conditioning product for the hair or a moisturising product for the skin.

When in the form of a cleansing product for the skin and hair, the cosmetic composition is preferably in the form of shampoo, conditioning shampoo (2-in-1), bath foam.

According to a preferred embodiment, the cosmetic composition in the form of a skin and hair cleanser comprises the salt of formula (I) in a concentration comprised between about 1 and 3%.

When in the form of a hair conditioning product, the cosmetic composition is in the form of an oil non oil conditioner or hair conditioner.

According to a preferred embodiment, the cosmetic composition in the form of a hair conditioning product comprises the salt of formula (I) in a concentration comprised between about 1 and 7%.

When in the form of a skin moisturising product, the cosmetic composition is preferably in the form of a moisturising cream.

According to a preferred embodiment, the cosmetic composition in the form of a skin moisturising cream comprises the salt of formula (I) in a concentration comprised between 1 and 3%.

When used as the sole cationic surfactant in hair cosmetic compositions, the quaternary ammonium salt of formula (I) shows comparable or more advantageous effects than common surfactants (see section Example 2).

According to a preferred embodiment, the cosmetic composition is in the form of a hair conditioning product and preferably comprises a fatty alcohol with a number of carbon atoms comprised between 14 and 22, preferably comprised between 16 and 18.

Preferably, the fatty alcohol contained in the composition is chosen from the group consisting of cetyl alcohol, stearyl alcohol or a combination of the foregoing (cetylstearyl alcohol). According to a preferred embodiment, the amount of alcohol is comprised between 1.10% and 2.20% by weight with respect to the amount of salt of formula (I).

The association with fatty alcohols advantageously promotes the combability, reducing the force needed for the operation of combing the hair with a smoother comb passage.

### Composition comprising the salt of formula (I) for surface treatment

The salt of formula (I), preferably of formula (I_{A}), is preferably used as a cationic surfactant.

Preferably, the salt of formula (I) is used as a wetting agent, conditioning/softening, emulsifying, cleaning, antistatic agent.

A further object of the present invention is a cleaning, softening/conditioning composition for surface treatment.

It should be noted that by surfaces it is intended both the household and non-household surfaces (objects, worktops, floors); leather surfaces; textile or fabric surfaces.

Preferably, the compositions for surface treatment that can be formulated with the quaternary ammonium salt of formula (I) are liquid compositions.

Preferably, the surface treatment comprises cleaning treatments of inert surfaces, leather or textiles, anti-static treatments of inert surfaces or textiles, softening treatments of textiles or leather, treatments for the care or maintenance of inert surfaces, leather or textiles and combinations of the foregoing.

In particular, the composition for surface treatment preferably comprises the salt of formula (I) in combination with any excipients and/or diluents.

It should be noted that diluents suitable for preparing compositions for surface treatment in accordance with the invention include:
- water: the compositions for surface treatment according to the present invention preferably contain water as the sole or main vehicle.
- solvents: they are used as washing additives or diluents in order to modulate the viscosity of the final product. Suitable solvents for the purposes of the invention are for example ethanol, glycerol, cetyl alcohol, fatty acids, monoglycerides, diglycerides and triglycerides.

It should be noted that organic solvents are generally used in the preparation of compositions for surface treatment when also synthetic surfactants are introduced in addition to the salt of formula (I). In order to make a composition for surface treatment green (hence that does not contain synthetic surfactants), the cetyl alcohol can be used as a solvent.

Composition for surface treatment comprising the salt of formula (I) according to the invention which preferably contains solvents in an amount comprised between 0 and 6% by weight on the total weight of the composition.

Composition for surface treatment comprising the salt of formula (I) according to the invention preferably containing water in an amount comprised between 0 and 95%, preferably between 0 and 90% by weight on the total weight of the composition.

Suitable excipients for the composition for surface treatment in accordance with the invention include, for example: chelators; amphoteric surfactants; cationic surfactants; pH control buffering systems; metal ion control agents; dyes; pigments; smell control agents (such as for example cyclodextrins); perfumes; essential oils; preservatives; anti-microbial agents; anti-mould agents; anti-oxidants; anti-corrosion agents; enzymes; water softening agents, bentonite, zeolite and combinations of the foregoing.

In the composition for surface treatment according to the invention preferably the salt of formula (I) is in a concentration comprised between about 0.25% and 7% (w/w), preferably between about 0.25% and 5%, preferably between about 0.5% and 5% (w/w), preferably between about 1% and 5% (w/w), preferably equal to about 0.25%, 0.5%, 1%, 2%, 3%, 4%, 5% (w/w) by weight on the total weight of the composition.

In the composition for surface treatment, the salt of formula (I) may be the only cationic surfactant or may be in combination with other cationic surfactants.

When used in combination with other cationic surfactants (e.g. polymeric cationic surfactants) or cationic/non-ionic surfactants or non-ionic surfactants, at the dosages indicated above, the salt of formula (I) allows the reduction of other polymeric cations.

Cationic surfactants that can be used in combination with the salt of formula (I) are, for example, chosen from the group consisting of: cetrimonium chloride (or CTAC), behentrimonium chloride (or BTAC), stearamidopropyl dimethylamine (or SAPDMA) and guar hydroxypropylthrimethylammonium chloride (or GHPTAC), Dioleylethyl Hydroxyethylmonium Methosulfate (Tetranil CO-40) and Behenoyl PG-Trimonium Chloride (Quartamina BTC-131).

In the composition for surface treatment, the salt of formula (I) can be used in combination with non-ionic surfactants. Non-ionic surfactants that can be used are preferably chosen among: polyoxyethylene derivatives of fatty acids and alkyl-polyglucosides or APGs, preferably they are APGs. Preferably, if the composition for surface treatment contains APGs, these may be generically chosen among ethanolamides, ethoxylated amides, ethoxylated amines, ethoxylated acids, cetomacrogol and mixtures thereof; preferably the non-ionic surfactant is chosen among Lauryl Polyglucoside, Cocamido propylamine oxide and mixtures of the foregoing.

The composition for surface treatment has preferably a pH higher than 3.5 and less than 6.0; preferably, the cosmetic composition has a pH comprised between 3.5 and 5, preferably comprised between 4 and 4.5.

Without wishing to be bound by any theory, the Applicant believes that the pH influences the conditioning/softening and anti-static capacities of a cationic system; in this sense, at the above pH values, the composition for surface treatment has shown improved conditioning/softening or anti-static capacities.

The pH of the composition for surface treatment can be regulated by including one or more buffering agents in the composition; an average person skilled in the art, on the basis of his basic knowledge, would be able to select without any difficulty one or more suitable buffering agents for the purpose, choosing among those known to be state of the art.

According to a preferred embodiment, the composition for surface treatment in the form of a detergent comprises the salt of formula (I) in a concentration comprised between 0.25% and 5%, preferably comprised between 0.25% and 2.5%, preferably comprised between 0.5% and 2%, preferably equal to 0.5%, 1%, 1.5% and 2% by weight, on the total weight of the composition (w/w).

When in the form of a conditioning/softening product, the composition comprises the salt of formula (I) in a concentration comprised between 0.5% and 7%, preferably comprised between 0.5% and 6%, preferably comprised between 1.5% and 5.5%, preferably equal to 2%, 2.5% 3%, 3.5%, 4%, 4.% 5% by weight, on the total weight of the composition (w/w).

### EXAMPLES

The Applicant provides hereinbelow examples for illustrative and non-limiting purposes of cosmetic use of the salt of formula (I).

### Example 1 - Examples of compositions comprising the salt of formula (I) for cosmetic use.

### 1.1 - Conditioning shampoo

| **Ingredients (INCI)** | **Basic shampoo A** | **Basic shampoo B** |
|---|---|---|
| Sodium Laureth Sulfate | 8.5 | 10.0 |
| Cocamidopropyl Betaine | - | 3.0 |
| Lauryl Hydroxysultanine | 3.0 | - |
| PEG-4 Rapeseedamide | - | 2.0 |
| Glicereth-2 Cocoate | 2.0 | - |
| **Salt of formula (I_{A1})** | 3.0 | 3.0 |
| Citric acid at pH 5 | qs | qs |
| Water | at 100 | at 100 |
| Preservative | q s | q s |
| Perfume | q s | q s |

### 1.2 - Conditioning shampoo (2-in-1)

| **Category** | **Ingredient (INCI)** | **% w/w** |
|---|---|---|
| Primary surfactant | Sodium Coceth-2 Sulphate | 12 |
| Auxiliary surfactants | Sodium Cocoamphoacetate | 5 |
| | Cocamidopropylamine Oxide | 3 |
| | Cocamidopropyl Betaine | 4 |
| Conditioners | **Salt of formula (I)** | 3 |
| | Guar Hydroxypropyltrimonium Chloride | 1 |
| | Hydroxypropyltrimonium Chloride | 1 |
| Solvent | Aqua (water) | at 100 |
| Active ingredients | Hydrolized Keratin | 1 |
| | Panthenol | 0.5 |
| Thickener | PEG-120 Methyl Glucose Trioleate, Propylen Glycol | 1 |
| Sequestrant | Tetrasodium EDTA | 0.1 |
| Perfume and preservatives | | q.s. |

### 1.3 - Green conditioning shampoo (2-in-1)

| **Category** | **Ingredient (INCI)** | **% w/w** |
|---|---|---|
| Primary surfactant | Sodium Lauroyl Glutamate | 12 |
| Auxiliary surfactants | Sodium Cocoamphoacetate | 5 |
| | Cocamidopropyl Hydroxysultanine | 3 |
| | Cocamidopropyl Betaine | 4 |
| Conditioners | **Salt of formula (I_{A1})** | 3 |
| | Distearoylethyl Hydroxyethylmonium Methosulfate | 1 |
| Solvent | Aqua (water) | at 100 |
| Active ingredients | Arctium Lappa Root Extract | 1 |
| | *Spirulina Maxima Powder* | 0.5 |
| Thickener | Xanthan Gum | 1 |
| Sequestrant | Tetrasodium Glutamate Diacetate | 0.1 |
| Perfume and preservatives | | q.s. |

### 1.4 - Hair conditioner

| **Category** | **Ingredient (INCI)** | **% w/w** |
|---|---|---|
| Emulsifier | Polyglyceryl-3 Cetyl Ether, Sesame Oil, Malic Acid | 4 |
| Surfactant | Cocamidopropyl Betaine | 3 |
| Thickeners/ stabilisers | Cetearyl Alcohol | 1.5 |
| | Myristyl Alcohol | 1 |
| Functional substances | Argan Oil | 1 |
| | Cetyl Dimethicone | 0.5 |
| Conditioners | **Salt of formula (I)** | 5 |
| | Behenamidopropyl Dimethylamine | 2 |
| Solvent | Aqua (water) | at 100 |
| Active ingredients | Phytantriol | 1 |
| | Sericin | 0.5 |
| | Urtica Dioica Leaf Extract | 1 |
| Sequestrant | Tetrasodium EDTA | 0.1 |
| Perfume and preservatives | | q.s. |

### 1.5 - 'Oil not oil' hair conditioner

| **Category** | **Ingredient (INCI)** | **% w/w** |
|---|---|---|
| Solubiliser | Polyglyceryl-6 Caprylate, Proline, Aqua | 3 |
| Fixative | VP/Dimethylaminoethylmethacrylate Copolymer | 2 |
| Solvent | Aqua (water) | at 100 |
| | Caprylyl Glycol | 3 |
| | Ethanol | 10 |
| Conditioner | **Salt of formula (I)** | 3 |
| | Polyquaternium-10 | 1 |
| Thickener | Methyl Gluceth-20 | 2 |
| Active ingredient | Panthenol | 1 |
| Sequestrant | Tetrasodium EDTA | 0.1 |
| Perfume and preservatives | | q.s. |

### 1.6 - After-bath body cream

| **Category** | **Ingredient (INCI)** | **% w/w** |
|---|---|---|
| Emulsifier | Polyglyceryl-6 Oleate, Potassium Olivate | 5 |
| Emollients | Persea Gratissima Oil | 3 |
| | Prunus Amygdalus Dulcis Oil | 3 |
| | Butyrospermum Parkii | 2 |
| | Caprylic/Capric Triglyceride | 5 |
| Thickeners/ Stabilisers | Glyceryl Stearate | 2 |
| | Cetyl Palmitate | 1 |
| | Cetearyl Alcohol | 1 |
| Functional substances | Sodium PCA | 1 |
| | Glycerin | 3 |
| | Urea | 0.5 |
| Conditioners | **Salt of formula (I)** | 5 |
| Solvent | Aqua (water) | at 100 |
| Active ingredients | Sodium Hyaluronate | 1 |
| | Bisabolol | 0.5 |
| | Hydrolyzed Mallow Leaf Extract | 1 |
| Sequestrant | Tetrasodium EDTA | 0.1 |
| Perfume and preservatives | | q.s. |

### Example 2 - Experimental evidence relative to the cosmetic use

### 2.1 - Visual assessment

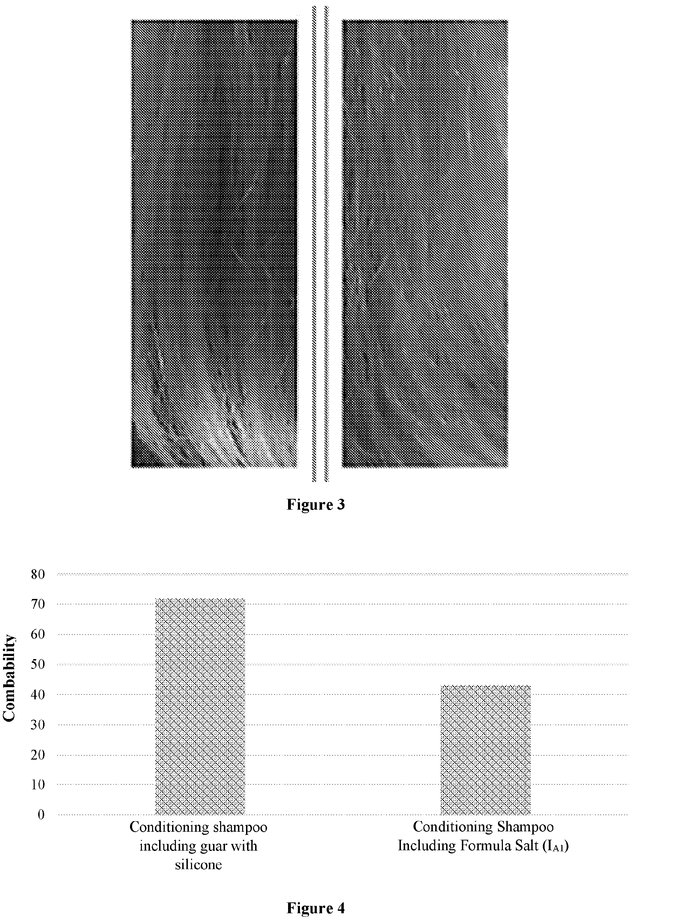

In the picture in Figure 3 it is possible to visually compare how a strand of hair treated with the cosmetic product comprising the salt of formula (I_{A1}) (on the left) looks like after drying and a strand of hair treated with a product comprising common quaternary cationic surfactants (quat) after drying (on the right).

### 2.2 - Method for measuring the combability

Caucasian hair strands of the same origin were washed for 1 minute with a shampoo and then treated for 1 minute with a hair conditioning product formulated with the quaternary ammonium salt of the invention and with conditioning products formulated with different cationic ingredients (control).

The combability measurements were then carried out with a tensiometer (INSTRON Mod. 5543, speed 500 mm/min) on both wet and dry hair (Figure 2).

In order to facilitate the interpretation of the results obtained by the Applicant, it should be noted that combability means the force that the hair opposes to the sliding of the comb, i.e. the measurement of the resistance that the hair exerts on the passage of the comb.

### 2.3 - Measurement of dry detangling of hair

The graph in Figure 4 shows the dry detangling values of damaged Caucasian hair, obtained by performing the method according to Example 2.2.

In particular, the hair was combed following the treatment with a conditioning shampoo and the conditioning shampoo of Example 1.1.

### 2.4 - Measurement of the detangling capacity on damaged Caucasian and Chinese hair

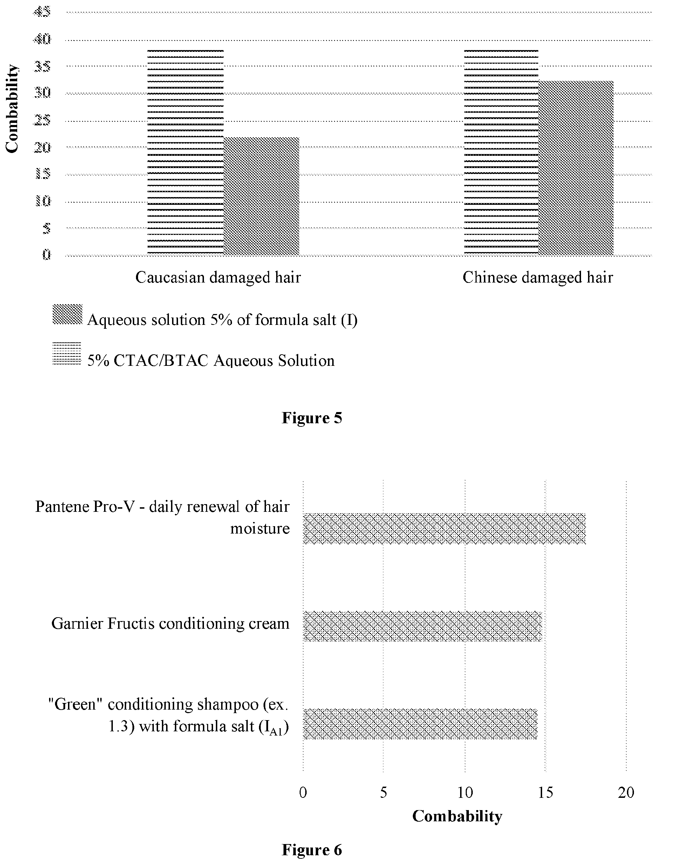

The graph in Figure 5 shows the dry detangling (combability) values of damaged Caucasian hair, obtained by performing the method according to Example 2.2.

In particular, the hair was combed following the treatment with a 5% aqueous solution of salt of formula (I_{A1}) and an equal amount of a 5% aqueous solution of a 50:50 combination of CTAC/BTAC.

The result of the treatment comprising the salt of formula (I_{A1}) shows better and significant benefits in the operation of dry hair combing (method in Example 2.2), if compared to the result obtained with the common quats, such as CTAC or BTAC.

In addition, the action of the salt of formula (I_{A1}) ensures a noticeable moisturising sensation on damaged hair.

### 2.5 - Hair thickness measurement assessed on five strands of hair after 20 brush strokes

The assessment was carried out on strands washed with the Green shampoo of example 1.3, compared with two traditional, market-leading products. The results are shown in the graph in Figure 6, expressed as a percentage increase in hair volume (before and after treatment).

Although the composition with the salt of formula (I_{A1}) is merely for illustrative purposes, the results are fully comparable with the complex and sophisticated compositions of products from major multinational companies containing synthetic ingredients.

### 2.6 - Hair breakage measurement after 10 brush strokes

The assessment was carried out on strands washed with the Green shampoo of example 1.3, compared with two traditional, market-leading used previously products (Control 1 and Control 2).

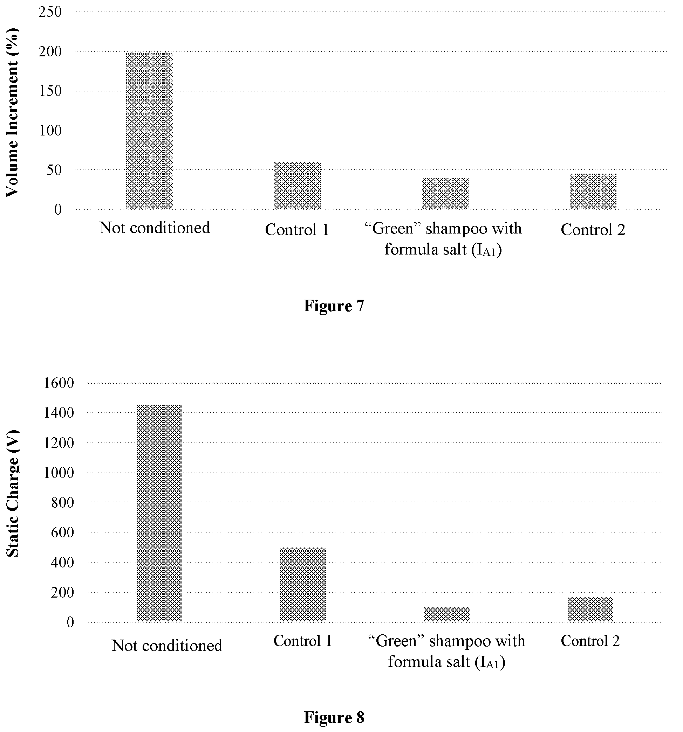

The results of this comparison can be seen in the graph in Figure 7.

### 2.7 - Method for measuring the electrostatic charge (static charge build-up - Lunn & Evans method).

The same strands of hair used for the combability tests were dried and combed 10 times under standard environmental conditions (20°C, relative humidity RH = 30%) and then the static charge (V) was measured with an appropriate sensor (3M Electical Specialties Division, Model 709).

The detection was carried out 10 times and the average of the values obtained was calculated.

From the results shown in the graph in Figure 8, it was found that a cosmetic product comprising the salt of formula (I_{A1}) acts as an effective static charge reducer.

The electrostatic charge abatement effect is higher when compared to that achieved with market-leading products comprising quaternary monoalkyl linear chains of CTAC and BTAC.

### Example 3 - Examples of compositions comprising the salt of formula (I) for surface treatment

### 3.1 - Composition in the treatment of leather, textile surfaces or fabrics

| **Salt of formula (I)** | 5-7% |
|---|---|
| Non-ionic surfactants (e.g. APG) | < 5% |
| Acidifier (e.g. citric acid) | q.s. |
| Sequestrant | q.s. |
| Perfume and preservatives | q.s. |
| Water | at 100 |

### 3.2 - Composition in the treatment of leather, textile surfaces or fabrics

| **Salt of formula (I)** | 5% |
|---|---|
| Acidifier (e.g. citric acid) pH 4 | q.s. |
| PERFUME | 0.5% |
| DYE | q.s. |
| PRESERVATIVE (MIT/BIT 5%) | 0.25% |
| Water | at 100 |

### 3.3 - Composition in the treatment of surfaces (objects, worktops, floors)

| | |
|---|---|
| APG (e.g. Lauryl Polyglucoside) | 4-5% |
| Cocamido propylamine oxide | 3-5% |

| **Salt of formula (I)** | 2-4% |
|---|---|
| Sequestrant (e.g. Tetrasodium Glutamate Diacetate) | q.s. |
| Perfume, preservatives and dyes | q.s. |
| Water | at 100 |

## Claims

1. Quaternary ammonium salt of formula (I) 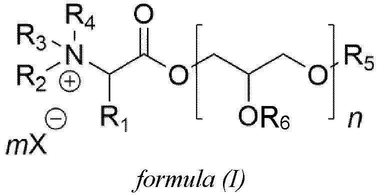 wherein the substituent R₅ is chosen between hydrogen and a radical R₀ wherein the radical R₀ consists of the following structure of formula (II) 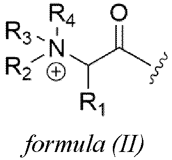
the substituent R₆ is hydrogen,
wherein the substituent R₁ is hydrogen,
wherein the substituents R₂, R₃, R₄ are methyl,
wherein the counteranion *m*X⁻ is chosen from the group of carboxylic acids consisting of: formic acid, acetic acid, unsaturated monocarboxylic acids, tartaric acid, adipic acid, aldaric acid, oxalic acid, phthalic acid, azelaic acid, sebacic acid, malonic acid, succinic acid, glutaric acid, pimelic acid, maleic acid, malic acid, fumaric acid, suberic acid, citric acid, isocitric acid, fatty acids, amino acids, keto acids and aromatic carboxylic acids,
wherein *m* is an integer number comprised between 1 and 22,
wherein *n* is comprised between 2 and 20.

2. Salt of formula (I) according to any one of claims from 1 to 4, wherein *n* is comprised between 2 and 10.

3. Salt of formula (I) according to claim 1, wherein *m* is an integer number comprised between 1 and 12.

4. Salt of formula (I) according to any one of claims from 1 to 3, wherein, R₅ is hydrogen, R₆ is hydrogen, *m* is equal to 1, and the counteranion X⁻ is the acetate ion or the malate ion or the citrate ion or the tartrate ion.

5. Salt of formula (I) according to any one of claims from 1 to 3, wherein R₅ is equal to R₀, R₆ is hydrogen, *m* is equal to 1 or 2, and the counteranion X⁻ is the acetate ion or the malate ion or the citrate ion or the tartrate ion or mixtures of the foregoing.

6. Composition comprising at least one salt of formula (I) according to any one of claims from 1 to 5, as a cationic surfactant.

7. Composition according to claim 6, wherein the salt of formula (I) is in a concentration comprised between about 0.25% and 7.00% (w/w) by weight on the total weight of the composition.

8. Composition according to claim 6 or 7, comprising the salt of formula (I) as the sole cationic surfactant or in combination with other cationic surfactants.

9. Composition according to any one of claims from 6 to 8, having a pH higher than 3.5 and less than 6.

10. Composition according to any one of claims from 6 to 9, the composition being a cosmetic composition.

11. Cosmetic composition according to claim 10, in the form of a skin and hair cleansing product, a hair conditioning product, a skin moisturising product.

12. Cosmetic composition according to claim 10 or 11, wherein the hair conditioning product comprises fatty alcohols with a number of carbon atoms comprised between 14 and 22.

13. Composition according to any one of claims from 6 to 9, the composition being a cleaning or conditioning/softening composition for surface treatment.

14. Composition according to claim 13, wherein the surface treatment is a treatment chosen from the group consisting of: cleaning treatments of inert surfaces, leather or textiles, anti-static treatments of inert surfaces or textiles, softening treatments of textiles or leather, treatments for the care or maintenance of inert surfaces, leather or textiles and combinations of the foregoing.

15. Composition according to claim 13 or 14, comprising, in addition to the salt of formula (I), suitable excipients and/or diluents.

## Patentansprüche

1. Quaternäres Ammoniumsalz der Formel **(I)** 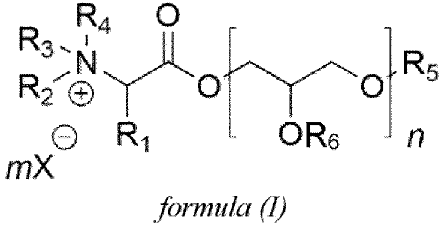 wobei der Substituent R₅ ausgewählt ist zwischen Wasserstoff und einem Rest Ro, wobei der Rest Ro aus der folgenden Struktur der Formel (II) besteht 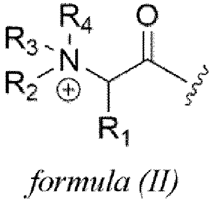
der Substituent *R₆*, Wasserstoff ist,
wobei der Substituent R₁ Wasserstoff ist,
wobei die Substituenten R₂, R₃, R₄ Methyl sind,
wobei das Gegenanion *mX⁻* ausgewählt ist aus der Gruppe der Carbonsäuren, bestehend aus: Ameisensäure, Essigsäure, ungesättigten Monocarbonsäuren, Weinsäure, Adipinsäure, Aldarsäure, Oxalsäure, Phthalsäure, Azelainsäure, Sebacinsäure, Malonsäure, Bernsteinsäure, Glutarsäure, Pimelinsäure, Maleinsäure, Äpfelsäure, Fumarsäure, Suberinsäure, Zitronensäure, Isocitronensäure, Fettsäuren, Aminosäuren, Ketosäuren und aromatischen Carbonsäuren,
wobei *m* eine ganze Zahl zwischen **1** und 22 ist,
wobei n zwischen 2 und 20 liegt.

2. Salz der Formel **(I)** nach einem der Ansprüche **1** bis 4, wobei *n* zwischen 2 und 10 liegt.

3. Salz der Formel (I) nach Anspruch 1, wobei m eine ganze Zahl zwischen 1 und 12 ist.

4. Salz der Formel (I) nach einem der Ansprüche 1 bis 3, wobei *Rs* Wasserstoff ist, R₆ Wasserstoff ist, m gleich 1 ist und das Gegenanion X⁻ das Acetat-Ion oder das Malat-Ion oder das Citrat-Ion oder das Tartrat-Ion ist.

5. Salz der Formel (I) nach einem der Ansprüche 1 bis 3, wobei *R*₅ gleich *R*₀ist, R₆ Wasserstoff ist, m gleich 1 oder 2 ist und das Gegenanion X⁻ das Acetat-Ion oder das Malat-Ion oder das Citrat-Ion oder das Tartrat-Ion oder Mischungen der Vorstehenden ist.

6. Zusammensetzung, umfassend mindestens ein Salz der Formel (I) nach einem der Ansprüche 1 bis 5 als kationisches Tensid.

7. Zusammensetzung nach Anspruch 6, wobei das Salz der Formel (I) in einer Konzentration zwischen etwa 0,25 und 7,00 Gew.-% (G/G), bezogen auf das Gesamtgewicht der Zusammensetzung, enthalten ist.

8. Zusammensetzung nach Anspruch 6 oder 7, umfassend das Salz der Formel (I) als alleiniges kationisches Tensid oder in Kombination mit anderen kationischen Tensiden.

9. Zusammensetzung nach einem der Ansprüche 6 bis 8, aufweisend einen pH-Wert von mehr als 3,5 und weniger als 6.

10. Zusammensetzung nach einem der Ansprüche 6 bis 9, wobei die Zusammensetzung eine kosmetische Zusammensetzung ist.

11. Kosmetische Zusammensetzung nach Anspruch 10, in Form eines Haut- und Haarreinigungsprodukts, eines Haarkonditionierungsprodukts, eines Hautbefeuchtungsprodukts.

12. Kosmetische Zusammensetzung nach Anspruch 10 oder 11, wobei das Haarkonditionierungsprodukt Fettalkohole mit einer Anzahl von Kohlenstoffatomen zwischen 14 und 22 umfasst.

13. Zusammensetzung nach einem der Ansprüche 6 bis 9, wobei die Zusammensetzung eine Reinigungs- oder Konditionierungs-/Weichspülungszusammensetzung zur Oberflächenbehandlung ist.

14. Zusammensetzung nach Anspruch 13, wobei die Oberflächenbehandlung eine Behandlung ist, die ausgewählt ist aus der Gruppe bestehend aus: Reinigungsbehandlungen von inerten Oberflächen, Leder oder Textilien, antistatischen Behandlungen von inerten Oberflächen oder Textilien, Weichmachungsbehandlungen von Textilien oder Leder, Behandlungen zur Pflege oder Wartung von inerten Oberflächen, Leder oder Textilien und Kombinationen der Vorstehenden.

15. Zusammensetzung nach Anspruch 13 oder 14, umfassend neben dem Salz der Formel (I) geeignete Hilfsstoffe und/oder Verdünnungsmittel.

## Revendications

1. Sel d'ammonium quaternaire de formule **(I)** 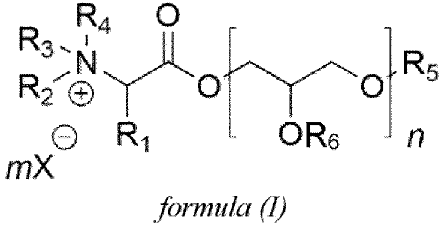 dans lequel le substituant R₅ est choisi entre l'hydrogène et un radical Ro dans lequel le radical Ro est constitué de la structure suivante de la formule (II) 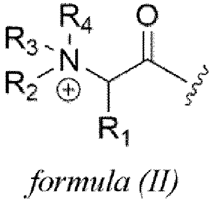
le substituant *R₆*, est l'hydrogène,
dans lequel le substituant R₁ est l'hydrogène,
dans lequel les substituants R₂, R₃, R₄ sont des méthyles,
dans lequel le contre-anion *mX⁻* est choisi dans le groupe des acides carboxyliques constitué de : acide formique, acide acétique, acides monocarboxyliques insaturés, acide tartrique, acide adipique, acide aldarique, acide oxalique, acide phtalique, acide azélaïque, acide sébacique, acide malonique, acide succinique, acide glutarique, acide pimélique, acide maléique, acide malique, acide fumarique, acide subérique, acide citrique, acide isocitrique, acides gras, acides aminés, cétoacides et acides carboxyliques aromatiques,
dans lequel *m* est un nombre entier compris entre **1** et 22,
dans lequel *n* est compris entre 2 et 20.

2. Sel de formule **(I)** selon l'une quelconque des revendications de **1** à 4, dans lequel *n* est compris entre 2 et 10.

3. Sel de formule (I) selon la revendication 1, dans lequel mis un nombre entier compris entre 1 et 12.

4. Sel de formule (I) selon l'une quelconque des revendications 1 à 3, dans lequel, *Rs* est l'hydrogène, R₆ est l'hydrogène, mis égal à 1, et le contre-anion X⁻ est l'ion acétate ou l'ion malate ou l'ion citrate ou l'ion tartrate.

5. Sel de formule (I) selon l'une quelconque des revendications 1 à 3, dans lequel *R*₅ est égal à R₀, R₆ est l'hydrogène, mis égal à 1 ou 2, et le contre-anion X⁻ est l'ion acétate ou l'ion malate ou l'ion citrate ou l'ion tartrate ou des mélanges de ce qui précède.

6. Composition comprenant au moins un sel de formule (I) selon l'une quelconque des revendications 1 à 5, en tant que tensioactif cationique.

7. Composition selon la revendication 6, dans laquelle le sel de formule (I) est dans une concentration comprise entre environ 0,25 % et 7,00 % (w/w) en poids sur le poids total de la composition.

8. Composition selon la revendication 6 ou 7, comprenant le sel de formule (I) comme seul agent de surface cationique ou en combinaison avec d'autres agents de surface cationiques.

9. Composition selon l'une quelconque des revendications 6 à 8, ayant un pH supérieur à 3,5 et inférieur à 6.

10. Composition selon l'une des revendications 6 à 9, la composition étant une composition cosmétique.

11. Composition cosmétique selon la revendication 10, sous forme de produit nettoyant pour la peau et les cheveux, de produit revitalisant pour les cheveux, de produit hydratant pour la peau.

12. Composition cosmétique selon la revendication 10 ou 11, dans laquelle le produit de conditionnement capillaire comprend des alcools gras dont le nombre d'atomes de carbone est compris entre 14 et 22.

13. Composition selon l'une des revendications 6 à 9, la composition étant une composition de nettoyage ou de conditionnement/adoucissement pour le traitement de surface.

14. Composition selon la revendication 13, dans laquelle le traitement de surface est un traitement choisi dans le groupe constitué par : les traitements de nettoyage des surfaces inertes, du cuir ou des textiles, les traitements antistatiques des surfaces inertes ou des textiles, les traitements d'assouplissement des textiles ou du cuir, les traitements d'entretien ou de maintenance des surfaces inertes, du cuir ou des textiles et les combinaisons de ce qui précède.

15. Composition selon la revendication 13 ou 14, comprenant, outre le sel de formule (I), des excipients et/ou des diluants appropriés.
